# EUROPEAN PATENT SPECIFICATION

(11) **EP 3 982 736 B1**
(45) Date of publication and mention of the grant of the patent: **08.10.2025**
(21) Application number: 20754057.6
(22) Date of filing: 12.06.2020
(51) Int. Cl.: A01N 63/30, C12N 1/14, A01N 25/08, A01N 25/04

(54) **INOCULUM FOR BIOTIZATION OF AMERICAN BLUEBERRY ITS PREPARATION AND USE THEREOF**
INOKULUM FÜR DIE BIOTISIERUNG VON AMERICAN BLUEBERRY SEINE ZUBEREITUNG UND VERWENDUNG
INOCULUM POUR LA BIOTISATION DU BLUEBERRY AMÉRICAIN SA PRÉPARATION ET SON UTILISATION

(30) Priority: 11.06.2019 PL 43020519
(43) Date of publication of application: 20.04.2022
(73) Proprietor: UNIWERSYTET JAGIELLONSKI, 31-007 Kraków (PL)
(72) Inventor: TURNAU, Katarzyna, 30-095 Kraków (PL); WAZNY, Rafal, 30-629 Kraków (PL); ROZPADEK, Piotr, 32-566 Poreba Zegoty (PL)
(74) Representative: Witek, Rafal
(86) International application number: PCT/PL2020/050048
(87) International publication number: WO 2020/251379

(56) References cited:
- CN-A- 102 876 584
- CN-A- 108 117 998
- WEZOWICZ KATARZYNA ET AL: "The diversity of endophytic fungi inVerbascum lychnitisfrom industrial areas", SYMBIOSIS, BALABAN PUBLISHERS, REHOVOT, ISRAEL, vol. 64, no. 3, 30 January 2015 (2015-01-30), pages 139 - 147, XP035454001, ISSN: 0334-5114, [retrieved on 20150130], DOI: 10.1007/S13199-015-0312-8
- MARTIN VOHNÃK ET AL: "Inoculation with a ligninolytic basidiomycete, but not root symbiotic ascomycetes, positively affects growth of highbush blueberry (Ericaceae) grown in a pine litter substrate ; Ligninolytic basidiomycete enhances growth of blueberry", PLANT AND SOIL ; AN INTERNATIONAL JOURNAL ON PLANT-SOIL RELATIONSHIPS, KLUWER ACADEMIC PUBLISHERS, DO, vol. 355, no. 1 - 2, 6 January 2012 (2012-01-06), pages 341 - 352, XP035061406, ISSN: 1573-5036, DOI: 10.1007/S11104-011-1106-2
- SIGURBJORN EINARSSON ET AL: "Production of Rhizobium Inoculants for Lupinus nootkatensis on Nutrient-Supplemented Pumice", APPLIED AND ENVIRONMENTAL MICROBIOLOGY, vol. 59, no. 11, 1 November 1993 (1993-11-01), pages 3666 - 3668, XP055112778

## Description

The invention relates to inoculum for biotization of American blueberry, comprising *Xylaria* sp. strain KKP 2073p mixed with strains of ericoid fungi, a method of preparing inoculum for biotization of American blueberry and a method of biotization of American blueberry. The objects of the invention are used in organic production of berries.

Biotization is a novel biotechnological approach to plant cultivation which involves inoculation a plant with useful microorganisms, such as fungi and bacteria, in order to increase its growth and tolerance to biotic and abiotic stress. Biotization of plants helps to reduce mineral fertilization of plants and increase the quantity and quality of crops.

From specification CN106635821 it is known to use mycorrhizal fungi to promote the absorption of nutrients, such as nitrogen and phosphorus, by American blueberry. Fungi were obtained through the isolation from roots of blueberries. The use of mycorrhizal fungi improved root development, promoted plant growth and the absorption of nitrogen, phosphorus and potassium.

From specification CN102220246 it is known to use mycorrhizal fungi *Coprinus micaceus,* isolated from American blueberry roots, to promote growth and improve the efficiency of American blueberry cultivation. The use of fungi *Coprinus micaceus* contributes to the improvement of root system of plants and protection against insects.

From specification WO2010050808 it is known to use ericoid fungi to improve soil quality. Strains of ericoid fungi belonging to *Hymenoscyphus ericae, Rhizoscyphus* spp. and *Oidiodendron* spp. are introduced into the soil together with silicate rock, which is a source of cations of magnesium, iron, sodium and others. The method improves soil quality for arable crops.

Publication of Wezowicz Katarzyna et al. "The diversity of endophylitic fungi in Verbascum lychnitis from industrial areas" in Symbiosis, vol. 64, no. 3. Pages 139-147 didsloses two *Xylaria endophytes* strains previously isolated from *Verbascum lychnitis* plants from industrial areas in Poland at Jagiellonian University and grow on PDA medium, but does not disclose quaternary combination according to subject invention.

Publication of Martin Vohnä K at al. "Inoculation with a ligninolytic basidiomycete, but not root symbiotic ascomycetes, positively affets growth of highbush blueberry (Ericaceae) grown in pine litter substrate; Ligninolytic basidiomycete enhances grown of blueberry" in Plant and Soil; An International Journal on Plant-Soil Relationships, vol. 355, no. 1-2, pages 341-352 discloses that basidiomycete fungi *A. preacos* fungi or its mixture with ericaceous fungi *O*. *maius*, *H.ericae*, *P.fortini* significantly increases the shoot growth of American blueberry on pine litter substrate whereas for ericaceous fungi alone no statistically significant difference could be observed, but does not disclose *Xylaria sp.*

Patent application CN108117998 (Zhejiang Academ Forestry) discloses that *Xylaria radix* strain promotes plant root growth and increases salt tolerance in plants, but does not disclose strain KKP 2073p.

Patent CN102876584 (Univ Zhejiang) discloses that *Xylaria striata* strain (isolated from wild rice) is useful for promoting growth of plants or for increasing biomass, but does not disclose strain KKP 2073p.

Publication of Sigurbjorn Einarsson et al. "Production of Rhisobium Inoculates for Lupinus nootkatensis on Nutrient-Supplemented Pumice", in Applied and Environmental Microbiology, vol. 59, no. 11, pages 3666-3668 discloses a growth medium for bacteria comprising wheat bran and diatomaceous earth, but does not disclose bacterial strains according to subject invention.The object of the present invention was to provide inoculum for biotization of American blueberry which contributes to the reduction of mineral fertilization of plants and, above all, increases the quality of American blueberry crops.

The inoculum for biotization of American blueberry according to the invention comprises a strain of genus *Xylaria* sp. No. KKP 2073p, deposited in the International Collection of Industrial Microorganisms of the Institute of Agricultural and Food Biotechnology in Warsaw under the number KKP 2073p,
and following strains of ericoid fungi:
- strain *Oidiodendron maius* UNIJAG.PL.368 deposited in the International Collection of Industrial Microorganisms of the Institute of Agricultural and Food Biotechnology in Warsaw under the number KKP 2077p,
- strain *Phialocephala fortinii* UNIJAG.PL.382 deposited in the International Collection of Industrial Microorganisms of the Institute of Agricultural and Food Biotechnology in Warsaw under the number KKP 2075p,
- strain *Hymenoscyphus sp.* UNIJAG.PL.391 deposited in the International Collection of Industrial Microorganisms of the Institute of Agricultural and Food Biotechnology in Warsaw under the number KKP 2076p.

The strain of genus *Xylaria* sp. No. KKP 2073p was isolated from the plant *Verbascum lychnitis.* The results of the genetic analysis of the ITS region of rDNA are presented in SEQ ID NO.1.

The method of preparing an inoculum for biotization of American blueberry according to the invention consists in that a strain of genus *Xylaria* sp. No. KKP 2073p, deposited in the International Collection of Industrial Microorganisms of the Institute of Agricultural and Food Biotechnology in Warsaw under the number KKP 2073p, is multiplied in a liquid carrier, particularly in 2% maltose medium, for a period of at least 5 days, and preferably of 7 days, after which the mycelium is separated from the medium and mixed with following strains of ericoid fungi:
- strain *Oidiodendron maius* UNIJAG.PL.368 deposited in the International Collection of Industrial Microorganisms of the Institute of Agricultural and Food Biotechnology in Warsaw under the number KKP 2077p,
- strain *Phialocephala fortinii* UNIJAG.PL.382 deposited in the International Collection of Industrial Microorganisms of the Institute of Agricultural and Food Biotechnology in Warsaw under the number KKP 2075p,
- strain *Hymenoscyphus sp.* UNIJAG.PL.391 deposited in the International Collection of Industrial Microorganisms of the Institute of Agricultural and Food Biotechnology in Warsaw under the number KKP 2076p.

Preferably, each strain comprised in the inoculum is multiplied separately on a suitable carrier, and then the strains are mixed with strain *Xylaria* sp. No. KKP 2073p in a specific quantity ratio before inoculation of American blueberry.

In the case of strains *Oidiodendron maius* UNIJAG.PL.368, UNIJAG.PL.382 and *Hymenoscyphus* sp. UNIJAG.PL.391, the carrier for multiplication of the strain are wheat bran with an admixture of diatomaceous earth, preferably in a volume proportion of 4:1.

Preferably, bran wheat with an admixture of diatomaceous earth should be sterile.

Preferably, strains *Oidiodendron maius* UNIJAG.PL.368, *Phialocephala fortinii* UNIJAG.PL.382 and *Hymenoscyphus* sp. UNIJAG.PL.391 are multiplied in the dark, at a temperature between 20°C and 30°C, preferably at 24°C, for a period of at least 1 month.

The method of biotization of American blueberry according to the invention is characterized in that American blueberry is inoculated with the inoculum according to the invention as defined above.

Preferably, American blueberry is inoculated when grown in vitro.

Preferably, blueberry is inoculated during the transfer from an in vitro culture to multiplates.

Preferably, American blueberry is inoculated during the transfer from multiplates to larger pots.

Preferably, inoculation is performed in the case of plants not older than one year of age.

Preferably, the inoculum is added to a sterile or a non-sterile substrate, wherein the substrate is preferably acidic peat with a pH ranging from 3.5 to 4.5 mixed with perlite, more preferably acidic peat with a pH ranging from 3.5 to 4.5 is mixed with perlite in a volume ratio of 5:1.

*Hymenoscyphus* sp. (strain UNIJAG.PL.391) was isolated from American blueberry variety Bluehaven, *Oidiodendron maius* (strain UNIJAG.PL.368) - variety P/14, *Phialocephala fortinii* (strain UNIJAG.PL.382) - variety Pliszka. Plants for isolation of symbionts came from American blueberry plantations, characterized by optimal growth conditions (peat medium with an acid reaction). Roots with the surrounding soil were collected from each plant at random. Next, the roots were washed under tap water and placed in dishes. Initial assessment of mycorrhizal colonization was performed with the use of a binocular at 10-40x magnification. Selected root hairs were placed on a basic slide and intended for microscopic observations at 100-400x magnification. Root fragments with visible mycelium threads in the cells were intended for isolation of fungi. The roots were surface sterilized for 50 seconds in 75% ethanol and then washed 3x in sterile water. After drying on sterile tissue paper, the roots were cut into fragments approximately 3 mm long and placed on drops of GelGro medium. The dishes were incubated in the dark at 27 °C. Fungi cultures were successively transferred to PDA medium and grown in the dark at 27°C.

Species identification was carried out based on microscopic observations and with the use of molecular biology techniques (amplification and sequencing of the ITS rDNA region). About 50 mg of mycelium originating from pure cultures was collected and homogenised for DNA extraction. DNA isolation was performed with the use of the EZ-10 Spin Column Fungal Genomic DNA Mini-Preps Kit reagent kit (Bio Basic, Canada). The content and purity of DNA was verified with the use of a spectrophotometric method. In the case of low efficiency of DNA isolation with the use of the above-mentioned method, the DNA was extracted again with the use of DNeasy Plant Mini Kit reagents (Qiagen) or the traditional method using CTAB. Impure extracts were purified with the use of 5M NaCl and isopropanol. The ITS rDNA fragment was then amplified by PCR using the ITS1F primers [Gardes and Bruns 1993] and ITS4 [White et al. 1990] and the use of Maxima Hot Start Green PCR Master Mix reagents (Thermo Scientific). The PCR parameters were as follows: initial denaturation at 95°C for 3 minutes; 35 cycles including, successively, denaturation at 95°C for 30 seconds, annealing at 55°C for 30 seconds and elongation at 72°C for 45 seconds and final elongation at 72°C for 5 minutes. The presence of PCR products was verified by agarose electrophoresis. The PCR products were sequenced by Macrogen with the use of the ITS4 and ITS1 primers. Nucleotide sequences were analyzed with the use of Chromas [www.technelysium.com.au] and BioEdit program and were compared with the sequences in the NCBI database [www.ncbi.nlm.nih.gov] with the use of the BLASTn algorithm. The results of molecular identification are presented in Table 1.

**Table 1. The results of molecular identification of ericoid fungi**

| **Strain** | **Identification** | **Reference Sequence (NCBI)** | **Similarity** |
|---|---|---|---|
| UNIJAG.PL.368 | *Oidiodendron maius* | *Oidiodendron maius* KF359579.1 | 552/557 (99%) |
| UNIJAG.PL.382 | *Phialocephala fortinii* | *Phialocephala fortinii* AY394921.1 | 551/553(99%) |
| UNIJAG.PL.391 | *Hymenoscyphus* sp. | *Hymenoscyphus* sp. EU700265.1 | 449/460 (98%) |

The results of the genetic analysis of the ITS rDNA segment are presented in SEQ ID NO.2 (strain UNIJAG.PL.368), SEQ ID NO.3 (strain UNIJAG.PL.382), SEQ ID NO.4 (strain UNIJAG.PL.391).

The method of biotization of American blueberry according to the invention consists in that American blueberry is inoculated with a strain of genus *Xylaria* sp. No. KKP 2073p, deposited in the International Collection of Industrial Microorganisms of the Institute of Agricultural and Food Biotechnology in Warsaw under the number KKP 2073p.

Preferably, American blueberry is inoculated when grown *in vitro.*

Preferably, American blueberry is inoculated during the transfer from an *in vitro* culture to multiplates.

The American blueberry is most effectively inoculated with the inoculum when transferring plants from multiplates to larger pots.

The inoculation is most effective in case of plants not older than one year of age.

The inoculum may be added to a sterile or a non-sterile substrate. In the roots of non-inoculated plants growing on a non-sterile substrate, mycorrhizal structures may appear, whose source are mycelium and ericoid fungal spores naturally occurring in such asubstrate.

The medium may be acidic peat with a pH ranging from 3.5 to 4.5, mixed with perlite.

The most effective medium is acidic peat with a pH ranging from 3.5 to 4.5, mixed with perlite in a volume ratio of 5:1.

The inoculum and the method of preparing the inoculum and the method of biotization of American blueberry according to the invention have great potential of commercial application in the production of planting material, contributing to the reduction of mineral fertilization of plants and, above all, to the increase of the quality of the crop.

An advantage of the invention is that each strain constituting the composition of the inoculum is grown separately in *in vitro* conditions on a specially developed carrier. Combination of all vaccine components occurs just before plant inoculation. Due to the fact that all organisms grow separately, the certainty of the presence of the living mycelium of each vaccine strain increases, which is an advantage over the existing solutions on the market so far, in the case of which there is no certainty as to the viability of all inoculum components.

The inoculum according to the invention is proposed for use in the cultivation of American blueberry, in particular for the varieties: Bluecrop, Duke, Dorrow and Patriot.

### Example I

The inoculum consists of the following strains of fungi: *Hymenoscyphus* sp. (strain UNIJAG.PL. *391), Oidiodendron maius* (strain UNIJAG.PL. 368), *Phialocephala fortinii* (strain UNIJAG.PL. 382) and strain of the genus *Xylaria* sp. No. KKP 2073p in a quantity ratio of 1:1:1:1.

Individual strains of fungi were prepared separately on developed carriers. For *Hymenoscyphus* sp., *O*. *maius* and *P. fortinii,* the mycelium carrier was wheat bran with an admixture of diatomaceous earth in a volume ratio of 4:1. The carrier of a volume of 50 ml was placed in a jar intended for an *in vitro* culture with the capacity of 200 ml and subjected to sterilization at 121°C for 30 min. Then, in sterile conditions, sterile water was added and 3 mycelia discs from a several-days-old culture on a solid PDA medium were placed on the carrier.

The inoculum was multiplied in the dark at 24°C for the period of at least 1 month. Before using the inoculum, 20-30 ml sterile water was added to the jar and shaken in order to reduce exposure to fungal spores during plant inoculation.

The strain of genus *Xylaria* sp. No. KKP 2073p was multiplied in liquid cultures in 2% maltose medium for the period of about 7 days. The mycelium was then filtered, rinsed with sterile water, fragmented and suspended in sterile water. The liquid inoculumcontaining the new strain of genus *Xylaria* sp. No. KKP 2073p was applied after the addition of the vaccine containing *Hymenoscyphus* sp. (Strain UNIJAG.PL.391), *Oidiodendron maius* (strain UNIJAG.PL.368), *Phialocephala fortinii* (strain UNIJAG.PL.382).

Blueberry plants 'Bluecrop' were inoculated at the stage of transfer from the *in vitro* culture to multiplates with peat. The dose of solid inoculum per plant was 1-2 ml of inoculum placed in a planting hole or mixed with the medium. Plants were watered after planting. The inoculum was added to the substrate, which was a non-sterile acidic peat with pH 3.5-4.5 mixed with perlite in a volume ratio of 5:1.

In 'Bluecrop' blueberry inoculated during transfer from the *in vitro* culture to peat substrate in multiplates, a significantly positive effect of the inoculum on fresh plant weight (increase by 60%), dry weight (increase by 64%), leaf surface (increase by 100%) was observed. In addition, plant vitality determined on the basis of chlorophyll fluorescence analysis in these plants was better than in control plants (27% increase in the Performance Index Abs).

### Example II

The inoculum consists of the following strains of fungi: *Hymenoscyphus* sp. (strain UNIJAG.PL. *391), Oidiodendron maius* (strain UNIJAG.PL. 368), *Phialocephala fortinii* (strain UNIJAG.PL. 382) and strain of the genus *Xylaria* sp. No. KKP 2073p in a quantity ratio of 1:1:1:1.

Individual strains of fungi were prepared separately on developed carriers. For *Hymenoscyphus* sp., *O*. *maius* and *P. fortinii*, the mycelium carrier was wheat bran with an admixture of diatomaceous earth in a volume ratio of 4:1. The carrier of a volume of 50 ml was placed in a jar intended for an *in vitro* culture with the capacity of 200 ml and subjected to sterilization at 121°C for 30 min. Then, in sterile conditions, sterile water was added and 3 mycelia discs from a several-days-old culture on a solid PDA medium were placed on the carrier.

The inoculum was multiplied in the dark at 24°C for the period of at least 1 month. Before using the inoculum, 20-30 ml sterile water was added to the jar and shaken in order to reduce exposure to fungal spores during plant inoculation.

The strain of genus *Xylaria* sp. No. KKP 2073p was multiplied in liquid cultures in 2% maltose medium for the period of about 7 days. The mycelium was then filtered, rinsed with sterile water, fragmented and suspended in sterile water. The liquid inoculum containing the new strain of genus *Xylaria* sp. No. KKP 2073p was applied after the addition of the inoculum containing *Hymenoscyphus* sp. (strain UNIJAG.PL.391), *Oidiodendron maius* (strain UNIJAG.PL.368), *Phialocephala fortinii* (strain UNIJAG.PL.382).

Blueberry plants 'Duke' were inoculated at the stage of transfer from multiplates to larger pots by placing inoculum in the substrate before repotting the plants. The dose of solid inoculum calculated for one plant transplanted into a 0.5-1 L pot was 5 ml of inoculum mixed with the substrate. Plants were watered after planting. The inoculum was added to the medium, which was a sterile acidic peat with pH 3.5-4.5 mixed with perlite in a volume ratio of 5:1. The use of sterile substrate (121°C, 30 min) required drying at 100°C (1h for 3 days) and misting on tissue paper for a period of 2 weeks.

Blueberries 'Duke' inoculated with the inoculum were characterized by a statistically higher height of the aboveground part (increase by 13%), above-ground biomass (increase by 21%) and leaf size (increase by 62%).

### Example III

The influence of a set of 3 most effective mycorrhizal symbionts in combination with saprobic/endophytic fungi was conducted.

For the inoculation of American blueberry, the strains of fungi most effective in previous experiments were selected:
- *Oidiodendron maius* (DNA strain 368)
- *Hymenoscyphus sp.* (DNA strain 391)
- *Phialocephala fortinii* (DNA strain 382)
and 2 saprobic/endophytic fungi:
- *Agrocybe praecox*
- *Xylaria sp.*

Ericoid fungi were grown on a carrier developed for the purpose of the project, based on bran and diatomaceous earth (mixed in a volume ratio of 40 ml:10 ml in a 200 ml *in vitro* culture jar) for two weeks at the temperature of 24°C in the dark. Approximately 2 ml of inoculum was placed in a planting hole.

Saprobic/endophytic fungi were grown in liquid cultures on maltose medium (2%). After 4 weeks, the cultures were rinsed with sterile tap water, placed in 100 ml sterile water and mixed. 1 ml of inoculum was applied to the planting hole for each fungi strain (sterile water was used in the control).

The experiment was carried out in polystyrene multiplates (with a capacity of 40 ml per 1 pot) on a substrate composed of light milled peat (0-40 mm fraction) with a pH (in H₂O) of 3.5-4.5 and perlite in a 1:5 ratio, sterilized in an autoclave at 121°C for 30 min and oven-dried for 3 consecutive days at 100°C for 1 hour. Plug trays with plants were placed in Sunbag bags (Sigma) in the growth chambers at 16h photoperiod and light intensity 90 µmol/cm² (60µmol/cm² from week 7 after inoculation), temperature 21/17°C. Each species of fungus was tested on 36 plants (3 multiplates x 12 plants).

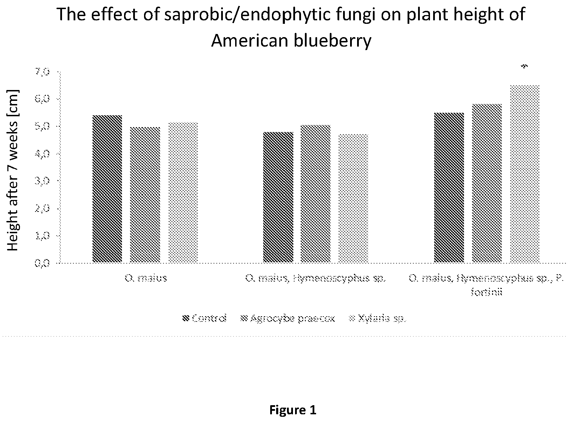

The effect of saprobic/endophytic fungi on plant height varied (Fig. 1). In plants inoculated with one or two species of ericoid fungi, no differences in height due to inoculation of saprobic fungi were observed, whereas in plants inoculated with *O*. *maius, Hymenoscyphus sp.* and *P. fortinii*, a positive effect of *Xylaria sp.* on height was observed.

## Claims

1. Inoculum for biotization of American blueberry, comprising:
- a strain of genus *Xylaria* sp. No. KKP 2073p, deposited in the International Collection of Industrial Microorganisms of the Institute of Agricultural and Food Biotechnology under the number KKP 2073p,
and following strains of ericoid fungi:
- strain *Oidiodendron maius* UNIJAG.PL.368 deposited in the International Collection of Industrial Microorganisms of the Institute of Agricultural and Food Biotechnology in Warsaw under the number KKP 2077p,
- strain *Phialocephala fortinii* UNIJAG.PL.382 deposited in the International Collection of Industrial Microorganisms of the Institute of Agricultural and Food Biotechnology in Warsaw under the number KKP 2075p,
- strain *Hymenoscyphus* sp. UNIJAG.PL.391 deposited in the International Collection of Industrial Microorganisms of the Institute of Agricultural and Food Biotechnology in Warsaw under the number KKP 2076p.

2. The method of preparing inoculum for biotization of American blueberry, **characterized in that** a strain of genus *Xylaria* sp. No. KKP 2073p, deposited in the International Collection of Industrial Microorganisms of the Institute of Agricultural and Food Biotechnology under the number KKP 2073p, is multiplied in a liquid medium, preferably in 2% maltose medium, for a period of at least 5 days, and preferably of 7 days, after which the mycelium is separated from the medium and mixed with following strains of ericoid fungi:
- strain *Oidiodendron maius* UNIJAG.PL.368 deposited in the International Collection of Industrial Microorganisms of the Institute of Agricultural and Food Biotechnology in Warsaw under the number KKP 2077p,
- strain *Phialocephala fortinii* UNIJAG.PL.382 deposited in the International Collection of Industrial Microorganisms of the Institute of Agricultural and Food Biotechnology in Warsaw under the number KKP 2075p,
- strain *Hymenoscyphus* sp. UNIJAG.PL.391 deposited in the International Collection of Industrial Microorganisms of the Institute of Agricultural and Food Biotechnology in Warsaw under the number KKP 2076p.

3. The method according to claim 2, **characterized in that** each strain comprised in the inoculum is multiplied separately on a suitable carrier, and then the strains are mixed with the strain of genus *Xylaria* sp. No. KKP 2073p in a specific quantity ratio before the inoculation of American blueberry.

4. The method according to claim 3, **characterized in that** in the case of strains *Oidiodendron maius* UNIJAG.PL.368, *Phialocephala fortinii* UNIJAG.PL.382 and *Hymenoscyphus* sp. UNIJAG.PL.391, the carrier for their multiplication are wheat bran with an admixture of diatomaceous earth, preferably in a volume proportion of 4:1.

5. The method according to claim 4, **characterized in that** wheat bran with the admixture of diatomaceous earth are sterile.

6. The method according to claim 4 or 5, **characterized in that** strains *Oidiodendron maius* UNIJAG.PL.368, *Phialocephala fortinii* UNIJAG.PL.382 and *Hymenoscyphus sp.* UNIJAG.PL.391 are multiplied in the dark, at a temperature between 20°C and 30°C, preferably at 24°C, for a period of at least 1 month.

7. A method of biotization of American blueberry, **characterized in that** American blueberry is inoculated with the inoculum according to claim 1.

8. The method according to claim 7, **characterized in that** American blueberry is inoculated when grown *in vitro.*

9. The method according to claim 7, **characterized in that** American blueberry is inoculated during the transfer from an *in vitro* culture to multiplates.

10. The method according to claim 7, **characterized in that** American blueberry is inoculated during the transfer from multiplates to larger pots.

11. The method according to claim 10, **characterized in that** inoculation is performed in the case of plants not older than one year of age.

12. The method according to any of the claims from 8 to 11, **characterized in that** the inoculum is added to a sterile or a non-sterile substrate, wherein the substrate is preferably acidic peat with a pH ranging from 3.5 to 4.5 mixed with perlite, more preferably acidic peat with a pH ranging from 3.5 to 4.5 is mixed with perlite in a volume ratio of 5:1.

## Patentansprüche

1. Inokulum für die Biotisierung der amerikanischen Heidelbeere, umfassend:
- einen Stamm der Gattung *Xylaria* sp. Nr. KKP 2073p, hinterlegt bei der International Collection of Industrial Microorganisms des Institute of Agricultural and Food Biotechnology unter der Nummer KKP 2073p,
und die folgenden Stämme von Ericoid-Mykorrhizapilzen:
- den Stamm *Oidiodendron maius* UNIJAG.PL.368, hinterlegt bei der International Collection of Industrial Microorganisms des Institute of Agricultural and Food Biotechnology in Warschau unter der Nummer KKP 2077p,
- den Stamm *Phialocephala fortinii* UNIJAG.PL.382, hinterlegt bei der International Collection of Industrial Microorganisms des Institute of Agricultural and Food Biotechnology in Warschau unter der Nummer KKP 2075p,
- den Stamm *Hymenoscyphus* sp. UNIJAG.PL.391, hinterlegt bei der International Collection of Industrial Microorganisms des Institute of Agricultural and Food Biotechnology in Warschau unter der Nummer KKP 2076p,

2. Verfahren zur Herstellung eines Inokulums für die Biotisierung der amerikanischen Heidelbeere, **dadurch gekennzeichnet, dass** ein Stamm der Gattung *Xylaria* sp. Nr. KKP 2073p, hinterlegt bei der International Collection of Industrial Microorganisms des Institute of Agricultural and Food Biotechnology unter der Nummer KKP 2073p, in einem flüssigen Medium, vorzugsweise in 2% Maltosemedium, über einen Zeitraum von wenigstens 5 Tagen und vorzugsweise 7 Tagen vermehrt wird, woraufhin das Myzel von dem Medium abgetrennt und mit den folgenden Stämmen von Ericoid-Mykorrhizapilzen gemischt wird:
- dem Stamm *Oidiodendron maius* UNIJAG.PL.368, hinterlegt bei der International Collection of Industrial Microorganisms des Institute of Agricultural and Food Biotechnology in Warschau unter der Nummer KKP 2077p,
- dem Stamm *Phialocephala fortinii* UNIJAG.PL.382, hinterlegt bei der International Collection of Industrial Microorganisms des Institute of Agricultural and Food Biotechnology in Warschau unter der Nummer KKP 2075p,
- dem Stamm *Hymenoscyphus* sp. UNIJAG.PL.391, hinterlegt bei der International Collection of Industrial Microorganisms des Institute of Agricultural and Food Biotechnology in Warschau unter der Nummer KKP 2076p.

3. Verfahren gemäß Anspruch 2, **dadurch gekennzeichnet, dass** jeder in dem Inokulum enthaltene Stamm separat auf einem geeigneten Träger vermehrt wird und dann die Stämme in einem speziellen Mengenverhältnis mit dem Stamm der Gattung *Xylaria* sp. Nr. KKP 2073p gemischt werden, bevor die amerikanische Heidelbeere angeimpft wird.

4. Verfahren gemäß Anspruch 3, **dadurch gekennzeichnet, dass** im Fall der Stämme *Oidiodendron maius* UNIJAG.PL.368, *Phialocephala fortinii* UNIJAG.PL.382 und *Hymenoscyphus sp.* UNIJAG.PL.391 es sich bei dem Träger für ihre Vermehrung um Weizenkleie mit einer Beimischung von Diatomeenerde, vorzugsweise in einem Volumenverhältnis von 4:1, handelt.

5. Verfahren gemäß Anspruch 4, **dadurch gekennzeichnet, dass** die Weizenkleie mit der Beimischung von Diatomeenerde steril ist.

6. Verfahren gemäß Anspruch 4 oder 5, **dadurch gekennzeichnet, dass** die Stämme *Oidiodendron maius* UNIJAG.PL.368, *Phialocephala fortinii* UNIJAG.PL.382 und *Hymenoscyphus sp.* UNIJAG.PL.391 im Dunkeln bei einer Temperatur zwischen 20°C und 30°C, vorzugsweise bei 24°C, über einen Zeitraum von wenigstens 1 Monat vermehrt werden.

7. Verfahren zur Biotisierung der amerikanischen Heidelbeere, **dadurch gekennzeichnet, dass** die amerikanische Heidelbeere mit dem Inokulum gemäß Anspruch 1 angeimpft wird.

8. Verfahren gemäß Anspruch 7, **dadurch gekennzeichnet, dass** die amerikanische Heidelbeere angeimpft wird, wenn sie *in vitro* wächst.

9. Verfahren gemäß Anspruch 7, **dadurch gekennzeichnet, dass** die amerikanische Heidelbeere während der Überführung von einer in-vitro-Kultur auf Multiplatten angeimpft wird.

10. Verfahren gemäß Anspruch 7, **dadurch gekennzeichnet, dass** die amerikanische Heidelbeere während der Überführung von Multiplatten auf größere Töpfe angeimpft wird.

11. Verfahren gemäß Anspruch 10, **dadurch gekennzeichnet, dass** die Impfung an Pflanzen in einem Alter von nicht mehr als einem Jahr durchgeführt wird.

12. Verfahren gemäß einem der Ansprüche 8 bis 11, **dadurch gekennzeichnet, dass** das Inokulum zu einem sterilen oder nichtsterilen Substrat hinzugefügt wird, wobei es sich bei dem Substrat vorzugsweise um sauren Torf mit einem pH-Wert im Bereich von 3,5 bis 4,5 handelt, der mit Perlit gemischt wurde, besonders bevorzugt saurer Torf mit einem pH-Wert im Bereich von 3,5 bis 4,5, der in einem Volumenverhältnis von 5:1 mit Perlit gemischt wird.

## Revendications

1. Inoculum pour la biotisation du myrtillier d'Amérique, comprenant :
- une souche du genre *Xylaria* sp. n° KKP 2073p, déposée à la Collection internationale de micro-organismes industriels de l'Institut de biotechnologie agricole et alimentaire sous le numéro KKP 2073p,
et les souches suivantes de champignons éricoïdes :
- la souche *Oidiodendron maius* UNIJAG.PL.368, déposée à la Collection internationale de micro-organismes industriels de l'Institut de biotechnologie agricole et alimentaire de Varsovie sous le numéro KKP 2077p,
- la souche *Phialocephala fortinii* UNIJAG.PL.382, déposée à la Collection internationale de micro-organismes industriels de l'Institut de biotechnologie agricole et alimentaire de Varsovie sous le numéro KKP 2075p,
- la souche *Hymenoscyphus* sp. UNIJAG.PL.391 déposée à la Collection internationale de micro-organismes industriels de l'Institut de biotechnologie agricole et alimentaire de Varsovie sous le numéro KKP 2076p.

2. Procédé de préparation d'inoculum pour la biotisation du myrtillier d'Amérique, **caractérisé en ce qu'**une souche du genre *Xylaria* sp. n° KKP 2073p, déposée à la Collection internationale de micro-organismes industriels de l'Institut de biotechnologie agricole et alimentaire sous le numéro KKP 2073p, est multipliée en milieu liquide, de préférence dans un milieu à 2 % de maltose, pendant une période d'au moins 5 jours, et de préférence de 7 jours, après quoi le mycélium est séparé du milieu et mélangé aux souches suivantes de champignons éricoïdes :
- souche Oidiodendron maius UNIJAG.PL.368, déposée à la Collection internationale de micro-organismes industriels de l'Institut de biotechnologie agricole et alimentaire de Varsovie sous le numéro KKP 2077p,
- souche *Phialocephala fortinii* UNIJAG.PL.382, déposée à la Collection internationale de micro-organismes industriels de l'Institut de biotechnologie agricole et alimentaire de Varsovie sous le numéro KKP 2075p,
- souche *Hymenoscyphus* sp. UNIJAG.PL.391, déposée à la Collection internationale de micro-organismes industriels de l'Institut de biotechnologie agricole et alimentaire de Varsovie sous le numéro KKP 2076p.

3. Procédé selon la revendication 2, **caractérisé en ce que** chaque souche de l'inoculum est multipliée séparément sur un support approprié, puis les souches sont mélangées à la souche du genre *Xylaria* sp. n° KKP 2073p dans un rapport de quantité spécifique avant l'inoculation du myrtillier d'Amérique.

4. Procédé selon la revendication 3, **caractérisé en ce que**, dans le cas des souches *Oidiodendron maius* UNIJAG.PL.368, *Phialocephala fortinii* UNIJAG.PL.382 et *Hymenoscyphus* sp. UNIJAG.PL.391, le support de leur multiplication est du son de blé additionné de terre de diatomées, de préférence dans un rapport volumique de 4:1.

5. Procédé selon la revendication 4, **caractérisé en ce que** le son de blé additionné de terre de diatomées est stérile.

6. Procédé selon la revendication 4 ou 5, **caractérisé en ce que** les souches *Oidiodendron maius* UNIJAG.PL.368, *Phialocephala fortinii* UNIJAG.PL.382 et *Hymenoscyphus* sp. UNIJAG.PL.391 sont multipliées à l'obscurité, à une température comprise entre 20 °C et 30 °C, de préférence à 24 °C, pendant une période d'au moins 1 mois.

7. Procédé de biotisation du myrtillier d'Amérique, **caractérisé en ce que** le myrtillier d'Amérique est inoculé avec l'inoculum selon la revendication 1.

8. Procédé selon la revendication 7, **caractérisé en ce que** le myrtillier d'Amérique est inoculé lors de la culture in vitro.

9. Procédé selon la revendication 7, **caractérisé en ce que** le myrtillier d'Amérique est inoculé lors du transfert d'une culture in vitro vers des boîtes multiplaques.

10. Procédé selon la revendication 7, **caractérisé en ce que** le myrtillier d'Amérique est inoculé lors du transfert des boîtes multiplaques vers des pots plus grands.

11. Procédé selon la revendication 10, **caractérisé en ce que** l'inoculation est réalisée sur des plantes âgées de moins d'un an.

12. Procédé selon l'une quelconque des revendications 8 à 11, **caractérisé en ce que** l'inoculum est ajouté à un substrat stérile ou non stérile, le substrat étant de préférence de la tourbe acide avec un pH compris entre 3,5 et 4,5 mélangée à de la perlite, de préférence encore de la tourbe acide avec un pH compris entre 3,5 et 4,5 mélangée à de la perlite dans un rapport volumique de 5:1.
